# EUROPEAN PATENT APPLICATION

(11) **EP 2 123 772 A1**
(43) Date of publication of application: **25.11.2009**
(21) Application number: 08155389.3
(22) Date of filing: 29.04.2008
(51) Int. Cl.: C12Q 1/18, C12Q 1/68, C07K 14/38, C07K 14/385, C12N 15/52, C12N 15/81, G01N 33/50

(54) **Beta-lactam antibiotic producing strains**

(71) Applicant: DSM IP Assets B.V., 6411 TE Heerlen (NL)
(72) Inventor: Berg, Van Den Marco Alexander, 2685 EH Poeldijk (NL); Bovenberg, Roelof Ary Lans, 3062 DA Rotterdam (NL); Driessen, Arnold Jacob Mattieu, 9727 DA GRONINGEN (NL); Woszczynska, Marta, 9753 AM Haren (NL)
(74) Representative: Misset, Onno

(57) **Abstract**

The present invention relates to a method for the identification of one or more genes of a parent microbial strain capable of producing a β-lactam compound and to mutant microbial strains in which the identified genes have been the functionally inactivated which results an at least 10% higher concentration of the total β-lactam compounds in the culture medium compared to the parent microbial strain and/or an at least 10% higher concentration of the desired β-lactam compound in the culture medium compared to the parent microbial strain; and/or an at least 10% improved yield (β-lactam / consumed sugar); and/or an at least 5% improved yield in desired β-lactam compound compared to undesired/total beta-lactam compound; and/or an at least 10% decreased consumption of side-chain precursor

## Description

### Field of the invention

The present invention relates to β-lactam compound producing strains, to a method for their construction as well to the identification and functional overexpression of genes and enzymes, leading to an increased production efficiency of β-lactam compounds.

### Background of the invention

Semi-synthetic β-lactam antibiotics (SSA's) are produced on an industrial scale starting from β-lactam compounds such as penicillinG (PenG), penicillinV (PenV), 6-aminopenicillanic acid (6-APA), 7-amino-desacetoxy-cephalosporanic acid (7-ADCA), 7-aminocephalosporanic acid (7-ACA) and 7-amino-3-chloro-3-cephem-4-carboxylate (7-ACCA), 7-amino-3-[(Z/E)-1-propen-1-yl]-3-cephem-4-carboxylate (7-PACA), 7-aminodeacetylcephalosporanic acid (7-ADAC), 7-amino-3-carbamoyloxymethyl-3-cephem-4-carboxylic acid (7-ACCCA) and others.

The production level of the β-lactam compounds in the commercially applied organisms has been increased considerably over the years. For instance, a modern *Penicillium chrysogenum* production strain is reported to produce about 40-50 g/I, whereas the original strains produced about 1 mg/l (Elander, R.P. (2002) University of Wisconsin contributions to the early development of penicillin and cephalosporin antibiotics SIM News 52, 270-278; Elander, R.P. (2003) Industrial production of β-/actam antibiotics. Appl Microbiol Biotechnol 61, 385-392). This enhanced production level was realised by classical mutagenesis techniques (Elander, R. (1983) Strain improvement and preservation of β-/actam producing microorganisms in A. L. Demain and N. Solomon (eds.) Antibiotics containing the β-/actam structure I. Springer-Verlag, New York, N.Y., 97-146).

PenicillinG can be used as a starting point to make semi-synthetic penicillins (SSP's) as amoxicillin and ampicillin, but it can also be used to make semi-synthetic cephalopsorins (SSC's). The first generation 7-ADCA product was derived from PenG whereby both the expansion of the 5-membered penem ring to the 6-membered cephem ring and the subsequent cleavage of the phenylacetic acid side chain of the phenylacetyl-7-ADCA were carried out using chemical reactions.

The next generation 7-ADCA product was still obtained from PenG but after the chemical ring expansion, the phenylacetic acid side chain of the phenylacetyl-7-ADCA was cleaved off enzymatically using a suitable (penicillin) acylase. Other processes have been developed wherein also the ring expansion of PenG to phenylacetyl-7-ADCA is carried out in vitro using a suitable expandase enzyme, but these processes are of little industrial importance. The most recent and most elegant production process for 7-ADCA comprises the culturing of a *Penicillium chrysogenum,* transformed with and expressing a gene encoding a suitable expandase. This engineered *Penicillium chrysogenum* strain, when grown in the presence of adipic acid as the side chain precursor in the fermentation vessel, produces and excretes adipoyl-7-ADCA - see WO93/05158. In this production process, the adipoyl-7-ADCA is recovered from the fermentation broth, subjected to a suitable acylase to cleave off the adipic acid side chain after which the 7-ADCA thus obtained is further purified, crystallized and dried. Other side chains precursors have been disclosed in WO95/04148 (2-(carboxyethylthio)acetic acid and 3-(carboxymethylthio)-propionic acid), WO95/04149 (2-(carboxyethylthio)propionic acid), WO96/38580 (phenyl acetic acid) and WO98/048034 and WO98/048035 (various dicarboxylic acids). The expandase takes care of the expansion of the 5-membered ring of the various N-acylated penicillanic acids, thereby yielding the corresponding N-acylated desacetoxycephalosporanic acids.

However, all these processes above rely on the efficiency of the host to convert the raw materials in to β-lactams. And although more than 60 years of classical strain improvement have increased this efficiency enormously, the titers and yields are far away from other classical fermentation processes like lysine (with *Corynebacterium glutamicum*) and ethanol (with *Saccharomyces cerevisae*), resulting in a large percentage of the expensive raw materials (i.e. carbon, nitrogen, but also side chain precursors like adipate, phenylacetic acid, etceteras) being lost in the form of biomass or CO₂.

It has now surprisingly found that specific genes in a parent microbial strain capable of producing a β-lactam compound may be functionally overexpressed which results in a more efficient production of the β-lactam compounds.

### Detailed description of the invention

Group 1 is defined herein as the group with gDNA sequences with SEQ ID No 1,. Table 1 summarizes the SEQ ID numbers together with their corresponding coding sequences and protein sequences as well as their gene ID.

In a first aspect, the invention provides a method for the identification of one or more genes of a parent microbial strain capable of producing a β-lactam compound comprising the steps of
a. Functionally inactivating the one or more genes in the parent microbial strain thereby generating one or more mutant microbial strains whereby each mutant microbial strain has at least one functionally inactivated gene;
b. Culturing the parent microbial strain and the mutant microbial strains obtained in step (a) in a medium under conditions that allow production of a β-lactam compound;
c. Measuring the concentration of the β-lactam compound in the culture medium of the parent microbial strain and the one or more mutant microbial strains;
d. Selecting the mutant microbial strains which possess one or more of the desired properties selected from the group consisting of
   (i) an at least 10% lower concentration of the total β-lactam compound in the culture medium compared to the parent microbial strain; and/or
   (ii) an at least 10% lower concentration of the desired β-lactam compound in the culture medium compared to the parent microbial strain; and/or
   (iii) an at least 10% lower yield (β-lactam / consumed sugar); and/or
   (iv) an at least 5% lower yield in desired beta-lactam compound compared to undesired/total beta-lactam compound; and/or
   (v) an at least 10% increased consumption of side-chain precursor.
e. Optionally identifying the gene(s) in the selected mutant microbial strain
f. Optionally repeating step a. - d. whereby a selected mutant strain obtained in step d. is used as the parent strain in step a.

In the context of the present invention, the genes identified by the method of the first aspect of the invention are referred to as *'positive genes'.*

"Functionally inactivating" or "functionally inactive" or "functional inactivation" or "functionally inactivated" is defined herein as the inactivation of a gene which results in a residual activity of the encoded enzyme as compared to the activity in the parent strain of preferably less than 50%, more preferably less than 40%, more preferably less than 30%, more preferably less than 20%, more preferably less than 10%, more preferably less than 5%, more preferably less than 2%.

A "parent microbial strain" may be defined as a micro-organism capable of producing β-lactam compounds, preferably N-adipoylated β-lactam compounds. A "mutant microbial strain" may be defined as a strain derived from the parent microbial strain by genetic engineering or classical mutagenesis.

The microbial strain capable of producing a β-lactam compound may be selected from the group consisting of a fungus, bacterium and yeast. Preferably the microbial strain of the present invention is a fungus, more preferably a filamentous fungus. A preferred filamentous fungus may be selected from the group consisting of *Aspergillus, Acremonium, Trichoderma* and *Penicillium.* More preferably the microbial strain of the present invention belongs to the species *Penicillium*, most preferably *Penicillium chrysogenum.* A preferred bacterium may be selected from the groups consisting of *Streptomyces, Nocardia,* or *Flavobacterium.* In a preferred embodiment, the microbial strain of the present invention capable of producing an N-adipoylated β-lactam compound belongs to the species *Penicillium*, most preferably is *Penicillium chrysogenum*, which has been transformed with a gene encoding an expandase, preferably the *Streptomyces clavuligerus cefE* gene, which enables the strain to produce adipoyl-7-ADCA when cultured in the presence of the precursor adipic acid. In another embodiment, the microbial strain of the present invention capable of producing an N-adipoylated β-lactam compound belongs to the species *Penicillium*, most preferably is *Penicillium chrysogenum* and which, in addition to an expandase gene, preferably the *Streptomyces clavuligerus cefE* gene, has been transformed with a hydroxylase gene, preferably the *Streptomyces clavuligerus cefF* gene, whose expression product converts the 3-methyl side chain of adipoyl-7-ADCA to 3-hydroxymethyl to give adipoyl-7-aminodeacetylcephalosporanic acid (adipoyl-7-ADAC). In another embodiment, the microbial strain of the present invention capable of producing an N-adipoylated β-lactam compound belongs to the species *Penicillium*, most preferably is *Penicillium chrysogenum*, has been transformed with a expandase/hydroxylase gene, preferably the *Acremonium chrysogenum cefEF* gene, whose expression product converts the 3-methyl side chain of adipoyl-7-ADCA to 3-hydroxymethyl, to give adipoyl-7-aminodeacetylcephalosporanic acid (adipoyl-7-ADAC). In another embodiment the microbial strain of the present invention capable of producing an N-adipoylated β-lactam compound belongs to the species *Penicillium*, most preferably is *Penicillium chrysogenum*, and in addition to the genes encoding expandase, preferably the *Streptomyces clavuligerus cefE* gene, and hydroxylase, preferably the *Streptomyces clavuligerus cefF* gene, is further transformed with an acetyltransferase gene, preferably the *Streptomyces clavuligerus cefG* gene, whose expression product (i.e. the acyltransferase) converts the 3-hydroxymethyl side chain to the 3-acetyloxymethyl side chain to give adipoyl-7-ACA. In a further embodiment the mutant microbial strain of the present invention capable of producing an N-adipoylated β-lactam compound belongs to the species *Penicillium*, most preferably is *Penicillium chrysogenum* and has been transformed with genes encoding an expandase, preferably the *Streptomyces clavuligerus cefE* gene, a hydroxylase, preferably the *Streptomyces clavuligerus cefF* gene and an O-carbamoyl transferase enzyme, preferably the *Streptomyces clavuligerus cmcH* gene, resulting in adipoyl-7-amino-3-carbamoyloxymethyl-3-cephem-4-carboxylic acid.

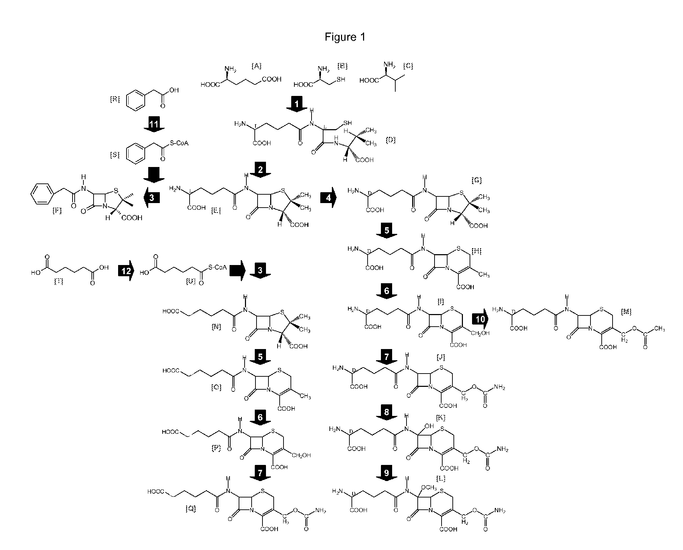

The β-lactam compound produced by the microbial strain may be any β-lactam wherein the β-lactam moiety is a penem or cephem. Preferred β-lactam compounds are those depicted in Figure 1. Most preferred β-lactam compounds are phenylacetyl- and adipoyl-derivates of the intermediates listed before: 6-aminopenicillanic acid (6-APA), 7-amino-desacetoxy-cephalosporanic acid (7-ADCA), 7-aminocephalosporanic acid (7-ACA) and 7-amino-3-chloro-3-cephem-4-carboxylate (7-ACCA), 7-amino-3-[(Z/E)-1-propen-1-yl]-3-cephem-4-carboxylate (7-PACA), 7-aminodeacetylcephalosporanic acid (7-ADAC), 7-amino-3-carbamoyloxymethyl-3-cephem-4-carboxylic acid (7-ACCCA) and others. Most preferred are N-adipoylated cephalosporins, most preferred is adipoyl-7-ADCA.

In step a) of the method of the invention, functionally inactivating the gene is defined as the modification of the gene in such a way so as to obtain a functionally inactive gene as defined hereinbefore. Methods for the modification of the gene in order to obtain the functionally inactive gene are known in the art and may include (but are not limited to): inactivation of the gene by base pair mutation resulting in a(n early) stop or frame shift; mutation of one or more codons which encode one or more a critical amino acids (such as the catalytic triad for hydrolases); mutations in the gene resulting in mutations in the amino acid sequence of the enzyme which lead to a decreased half-life of the enzyme; modifying the mRNA molecule in such away that the mRNA half-life is decreased; insertion of a second sequence (i.e. a selection marker gene) disturbing the open reading frame; a partial or complete removal of the gene; removal/mutation of the promoter of the gene; physical separation of promoter and gene by insertion of third DNA sequence (for example a selection marker gene); replacing the promoter of the gene by a regulatable promoter; using anti-sense DNA or comparable RNA inhibition methods to lower the effective amount of mRNA in the cell. Most preferably the gene may be made functionally inactive by deletion (as described in example 1) resulting in a total absence of the encoded polypeptide and hence enzyme activity.

One approach is a temporary one using an anti-sense molecule or RNAi molecule (Kamath et al. 2003. Nature 421:231-237). Another is using a regulatable promoter system, which can be switched off using external triggers like tetracycline (see Park and Morschhauser, 2005, Eukaryot Cell. 4:1328-1342). Yet another one is to apply a chemical inhibitor or a protein inhibitor or a physical inhibitor (see Tour et al. 2003. Nat Biotech 21:1505-1508). The most preferred method is to remove part of or the complete gene(s) encoding the enzyme directly or indirectly mediating the incorporation efficiency of raw materials into the β-lactam compound. To obtain such a mutant one can apply state of the art methods like Single Cross-Over Recombination or Double Homologous Recombination. For this, one needs to construct an integrative cloning vector that may integrate at the predetermined target locus in the chromosome of the host cell. In a preferred embodiment of the invention, the integrative cloning vector comprises a DNA fragment, which is homologous to a DNA sequence in a predetermined target locus in the genome of host cell for targeting the integration of the cloning vector to this predetermined locus. In order to promote targeted integration, the cloning vector is preferably linearized prior to transformation of the host cell. Linearization is preferably performed such that at least one but preferably either end of the cloning vector is flanked by sequences homologous to the target locus. The length of the homologous sequences flanking the target locus is preferably at least 0.1 kb, even preferably at least 0.2 kb, more preferably at least 0.5 kb, even more preferably at least 1 kb, most preferably at least 2 kb. The length that finally is best suitable in an experiment depends on the organism, the sequence and length of the target DNA., The efficiency of targeted integration of a nucleic acid construct into the genome of the host cell by homologous recombination, i.e. integration in a predetermined target locus, is preferably increased by augmented homologous recombination abilities of the host cell. Such phenotype of the cell preferably involves a deficient *hdfA* or *hdfB* gene as described in WO 05/95624. WO 05/95624 discloses a preferred method to obtain a filamentous fungal cell comprising increased efficiency of targeted integration by preventing non-homologous random integration of DNA fragments into the genome. The vector system may be a single vector or plasmid or two or more vectors or plasmids, which together contain the total DNA to be introduced into the genome of the host cell. Alternative methods using second and/or lethal selectable markers are described in WO2007115886 and WO2007115887. Fungal cells may be transformed by protoplast formation, protoplast transformation, and regeneration of the cell wall. Suitable procedures for transformation of fungal host cells are described in EP 238023 and Yelton et al. (1984. Proc. Nat. Acad. Sci. USA 81:1470-1474). Suitable procedures for transformation of filamentous fungal host cells using *Agrobacterium tumefaciens* are described by de Groot M.J. et al. (1998. Nat. Biotechnol. 16:839-842. Erratum in: Nat. Biotechnol. 1998. 16:1074). Other methods like electroporation, described for *Neurospora crassa,* may also be applied. Fungal cells are transfected using co-transformation, *i*.*e*. along with gene(s) of interest also a selectable marker gene is transformed. This can be either physically linked to the gene of interest (*i*.*e*. on a plasmid) or on a separate fragment. Following transfection transformants are screened for the presence of this selection marker gene and subsequently analyzed for the integration at the preferred predetermined genomic locus. A selectable marker is a product, which provides resistance against a biocide or virus, resistance to heavy metals, prototrophy to auxotrophs and the like. Useful selectable markers include, but are not limited to, *amdS* (acetamidase), *argB* (ornithinecarbamoyl-transferase), *bar* (phosphinothricinacetyltransferase), *hygB* (hygromycin phosphotransferase), *niaD* (nitrate reductase), *pyrG* (orotidine-5'-phosphate decarboxylase), *sC* or *sutB* (sulfate adenyltransferase), *trpC* (anthranilate synthase), ble (phleomycin resistance protein), as well as equivalents thereof. The most preferred situation is providing a DNA molecule comprising a first DNA fragment comprising a desired replacement sequence (i.e. the selectionmarker gene) flanked at its 5' and 3' sides by DNA sequences substantially homologous to sequences of the chromosomal DNA flanking the target sequence. Cells wherein the target sequence in the chromosomal DNA sequence is replaced by the desired replacement sequence can be selected by the presence of the selectable marker of the first DNA fragment. To increase the relative frequency of selecting the correct mutant microbial strain, a second DNA fragment comprising an expression cassette comprising a gene encoding a selection marker and regulatory sequences functional in the eukaryotic cell can be operably linked to the above described fragment (i.e. 5'-flank of target locus + selection marker gene + 3'-flank of target locus) and cells wherein the target sequence in the chromosomal DNA sequence is replaced by the desired replacement sequence can be selected by the presence of the selectable marker of the first DNA fragment and the absence of the second selection marker gene. The 5'- and 3'-flanks of the target locus can be for example the promoter and terminator of a gene, or the 5'- and 3'-end of the gene or any combination of these.

Another preferred method uses the promoter of the gene as 5'-flank and the gene as the 3'-flank to insert a selection marker between the promoter and gene, thereby disturbing (i.e. functionally inactivating) gene transcription. The gene sequences given above can be used to make similar functionally inactivated genes. The genes may be split in two, yielding a 5'-flank and a 3'-flank, but the gene may also be used to clone a larger piece of genomic DNA containing the promoter and terminator regions of the gene, which than can function as 5'-flank and a 3'-flanks.

In step b) of the method of the invention, the parent and mutant microbial strains capable of producing a β-lactam compound may be cultured in a medium which allows the production of said β-lactam. These methods are well known to the person skilled in the art. One way of culturing said strains is described in the Examples. Depending on the β-lactam to be produced, the culture medium may comprise a side chain precursor, e.g. adipic acid or a suitable salt thereof for the production of adipylated β-lactam compounds (e.g. adipyl-6-APA, adipyl-7-ADCA, adipyl-7-ACA and others) or phenyl acetic acid for the production of penicillin G or other benzyl β-lactam compounds and so on. The culture medium furthermore may comprise a suitable carbon source such as a sugar (glucose, lactose and others).

In step c) of the method of the invention, the concentration of the β-lactam compounds may be measured by methods well known to the person skilled in the art. In addition to the desired β-lactam compound, the microbial strains may also produce and excrete other so-called undesired β-lactam compounds. The sum of desired and undesired β-lactam compounds is referred to here as the total β-lactam compounds produced. Amongst the β-lactam compounds that may be measured are desired β-lactam compounds such as adipoyl-7-ADCA and penicillinG and undesired β-lactam intermediates or compounds such as L-α-aminoadipyl-L-cysteinyl-D-valine and isopenicillin as well as degradation products like 8-hydroxy-penillic-acid (see for an overview Figure 1). Other relevant concentrations that may be measured are the concentration of the carbon source such as sugar, the remaining concentration of the precursor as well as the concentration of degradation products of the precursor (for example ortho-hydroxyphenylacetic acid, OH-PAA). One way of measuring said concentrations is described in the Examples.

In step d) of the method of the invention, the mutant microbial strains capable of producing a β-lactam are selected for one or more of the desired properties cited. In one embodiment, mutant microbial strains are selected based on the concentration of the total β-lactam compounds produced in the culture medium under the conditions of the test. Preferably, the culture medium of the mutant microbial strain has an at least 10% lower concentration, more preferably an at least 20% lower concentration, more preferably an at least 30% lower concentration, more preferably an at least 40% lower concentration, more preferably an at least 50% lower concentration, more preferably an at least 75% lower concentration, more preferably an at least 90% lower concentration, more preferably an at least 95% lower concentration, more preferably an at least 99% lower concentration more preferably an at least 100% lower concentration of the total β-lactam compounds compared to the concentration of the total β-lactam compounds produced by the parent microbial strain.

In another embodiment, mutant microbial strains are selected based on the concentration of the desired β-lactam compound produced in the culture medium under the conditions of the test. Preferably, the culture medium of the mutant microbial strain has an at least 10% lower concentration, more preferably an at least 20% lower concentration, more preferably an at least 30% lower concentration, more preferably an at least 40% lower concentration, more preferably an at least 50% lower concentration, , more preferably an at least 75% lower concentration, more preferably an at least 90% lower concentration, more preferably an at least 95% lower concentration, more preferably an at least 99% lower concentration, most preferably an at least 100% lower concentration of the desired β-lactam compound compared to the concentration of the desired β-lactam compound produced by the parent microbial strain

In a further embodiment, mutant microbial strains capable of producing a β-lactam are selected based on an at least 10% lower yield of total β-lactam on consumed sugar compared to the yield of the parent microbial strain (expressed as percentage - In Table 1, this yield is referred to as Ybs), more preferably at least 20% lower yield, more preferably at least 30% lower yield, more preferably at least 40% lower yield, more preferably at least 50% lower yield, more preferably at least 75% lower yield, more preferably at least 90% lower yield, more preferably at least 95% lower yield, more preferably at least 99% lower yield, most preferably at least 100% lower yield compared to the yield of the parent microbial strain. This yield is defined herein as ratio of the amount or millimoles of total β-lactam compounds found in the culture medium and the amount of mole sugar consumed. The amount of total β-lactam compounds found in the culture medium may be calculated as follows:
(i) determining the molar concentration of each β-lactam compound in the culture medium and
(ii) multiplying the value of i. by 1000;
(iii) sum up all the individual values for each β-lactam compound.
The amount of Cmol consumed sugar is calculated as follows:
(i) determining the concentration of sugar in the medium at the start of the cultivation;
(ii) determining the concentration of sugar in the medium at the end of the cultivation;
(iii) substracting the value of i. from the value of ii.
(iv) dividing the value of iii. by the molecular weight of the sugar per Cmol (for glucose this is 30.03).

In another embodiment, mutant microbial strains capable of producing a β-lactam are selected based on at least 5% lower yield in desired β-lactam compound with respect to the total β-lactam compounds produced compared to the yield of the parent microbial strain. For instance, when the parent microbial strain produces out of the 100% total β-lactam compounds only 80% of the desired β-lactam, the yield of the parent β-lactam producing strain is by definition 80%. The mutant microbial strain with an at least 5% lower yield produces the desired β-lactam compound with a yield of 76% (80 - 0.05*80). Preferably the yield at least 5% lower, preferably at least 10%, more preferably at least 20%, more preferably at least 30%, more preferably at least 40%, more preferably at least 50%, more preferably at least 75%, more preferably at least 90%, more preferably at least 95%, more preferably at least 99%, most preferably 100% lower compared to the yield of the parent microbial strain.

In a further embodiment, mutant microbial strains are selected based on an increased consumption of precursor. It is well known in the art, that the precursors used for the production of the various β-lactam compounds (e.g. phenyl acetic acid, adipic acid etceteras), are not only incorporated in the desired β-lactam compounds, but may also be degraded via several metabolic routes for instance to serve as a carbon source to the microbial strain. Mutant microbial strains capable of producing a β-lactam are selected based on an at least 10% increased precursor consumption compared to the precursor consumption of the parent microbial strain. The increased consumption may be derived from the remaining concentration of the precursor after the fermentation step. Mutant microbial strains are selected on the basis of an at least 10% lower remaining concentration of the precursor, more preferably an at least 20% lower concentration, more preferably an at least 30% lower concentration, more preferably an at least 40% lower concentration, more preferably an at least 50% lower concentration, more preferably an at least 75% lower concentration, more preferably an at least 90% lower concentration, more preferably an at least 95% lower concentration, more preferably an at least 99% lower concentration, most preferably an at least 100% lower concentration of the precursor compared to the parent strain.

In step e) of the method of the invention, the gene that has been functionally inactivated in the selected mutant microbial strain may further be identified which may be necessary when the gene coding regions are not known at forehand. In a preferred embodiment however, part or the total of the genomic sequence of the parent β-lactam producing microbial strain may be determined. Analysis of the genomic sequences may then result in the determination of gene coding regions as well as the putative function of the enzymes encoded by the genes. The latter may be done by sequence comparisons with known gene- and/or protein sequences according to methods known in the art. In this embodiment it is possible to individually functionally inactivate one or more of at forehand selected, for instance based on the putative function, gene coding regions.

According to step f) of the method of the invention step a. - d. may be repeated one or several times whereby a selected mutant strain obtained in step d. of a previous round is used as the parent strain in step a. in a next round. Repeating steps a.-d. one or several times may increase the likelihood that the mutant β-lactam producing microbial strain of the invention may contain more than one functionally inactivated genes and, as a consequence, may further be decreased in one or more of the desired properties listed above.

In one embodiment, the method of the first aspect of the invention may also identify one or more genes of a parent microbial strain capable of producing a β-lactam compound that upon functionally inactivating the one or more genes in the parent microbial strain lead to an improvement of the desired properties defined hereinbefore, such as:
a. an at least 10% higher concentration of the total β-lactam compounds in the culture medium compared to the parent microbial strain; and/or
b. an at least 10% higher concentration of the desired β-lactam compound in the culture medium compared to the parent microbial strain; and/or
c. an at least 10% improved yield (β-lactam / consumed sugar); and/or
d. an at least 5% improved yield in desired β-lactam compound compared to undesired/total beta-lactam compound; and/or
e. an at least 10% improved yield in desired β-lactam on precursor (β-lactam /consumed precursor).
In the context of the present invention, these genes identified by the method of the first aspect of the invention are referred to as *'negative genes'.*

In a second aspect, the invention provides a gene identifiable by the method of first aspect of the invention. Preferably, the invention provides a gene selected from group 1 as defined hereinbefore. Table 1 summarizes the SEQ ID numbers of the genomic DNA sequences of the genes of the invention together with their corresponding coding sequences and protein sequences as well as their gene ID.

In a third aspect, the invention provides a polypeptide which is encoded by the gene of the second aspect of the invention. Preferably, the invention provides a polypeptide which is encoded by a gene selected from group 1 as defined hereinbefore and may be selected from the group consisting of SEQ ID No 3. Table 1 summarizes the SEQ ID numbers of the genomic DNA sequences of the genes of the invention together with their corresponding coding sequences and protein sequences as well as their gene ID.

It has surprisingly been found that "functional overexpresssion" one or more of the genes of the invention in a microbial strain capable of producing a β-lactam compound leads to an improvement of the desired properties as defined in the method of the first aspect of the invention. Therefore, the polypeptides encoded by the genes of the invention are 'involved' in the production of the β-lactam compound by the microbial strain. In the context of this invention 'involved' is used to cover the various functions by which a gene and/or a protein influence a certain phenotype, in this case the efficiency of incorporation of raw materials from the culture medium into β-lactam compounds. The effect can be obtained as a 'direct effect', i.e. the functionally overexpressed gene product is directly responsible for the observed phenotype; or as an 'indirect effect', i.e. the functionally overexpressed gene or gene product is causing modifications in other gene or gene products. These include, but are not limited to: improved import of the raw materials; improved conversion of raw materials to β-lactam building blocks; improved conversion of the β-lactam into β-lactam compounds; improved intracellular transport of the building blocks; improved intracellular transport of the β-lactam compounds; decreased degradation of the building blocks; decreased degradation of the β-lactam compounds; increased volumes and/or numbers of organels; increased number of proteins; modification of the membrane structure; modification of hyphal differentiation; modification of the ploidy level.

The amino acid sequences are not limited to the sequences listed above but also comprise sequences that are "substantially homologous" to said sequences with the proviso that the polypeptide having such an amino acid sequence is 'involved' in the production of β-lactam compounds and upon functional overexpresssion leads to an improvement of the desired properties defined in the first aspect.

For the purpose of the present invention, the homology between two amino acid sequences refers to the percentage of amino acids that are identical between the two sequences. The degree of identity between two amino acid sequences refers to the percentage of amino acids that are identical between the two sequences.

The degree of identity between two amino acid sequences refers to the percentage of amino acids that are identical between the two sequences. The degree of identity is determined using the BLAST algorithm, which is described in Altschul et al. (J. Mol. Biol. 215: 403-410 (1990)). Software for performing BLAST analyses is publicly available through the National Center for Biotechnology Information (http://www.ncbi.nlm.nih.gov/). The BLAST algorithm parameters W, T, and X determine the sensitivity and speed of the alignment. The BLAST program uses as defaults a word length (W) of 11, the BLOSUM62 scoring matrix (see Henikoff & Henikoff, Proc. Natl. Acad. Sci. USA 89: 10915 (1989)) alignments (B) of 50, expectation (E) of 10, M=5, N=-4, and a comparison of both strands. A substantially homologous polypeptide may encompass polymorphisms that may exist in cells from different populations or within a population due to natural allelic or intra-strain variation. A substantially homologous polypeptide may further be derived from a fungus other than the fungus where the specified amino acid and/or DNA sequence originates from, or may be encoded by an artificially designed and synthesized DNA sequence. DNA sequences related to the specified DNA sequences and obtained by degeneration of the genetic code are also part of the invention. Homologues may also encompass biologically active fragments of the full-length sequence. Substantially homologous polypeptides may contain only conservative substitutions of one or more amino acids of the specified amino acid sequences or substitutions, insertions or deletions of non-essential amino acids. Accordingly, a non-essential amino acid is a residue that can be altered in one of these sequences without substantially altering the biological function. For example, guidance concerning how to make phenotypically silent amino acid substitutions is provided in Bowie, J.U. et al., (Science 247:1306-1310 (1990)) wherein the authors indicate that there are two main approaches for studying the tolerance of an amino acid sequence to change. The first method relies on the process of evolution, in which mutations are either accepted or rejected by natural selection. The second approach uses genetic engineering to introduce amino acid changes at specific positions of a cloned gene and selects or screens to identify sequences that maintain functionality. As the authors state, these studies have revealed that proteins are surprisingly tolerant of amino acid substitutions. The authors further indicate which changes are likely to be permissive at a certain position of the protein. For example, most buried amino acid residues require non-polar side chains, whereas few features of surface side chains are generally conserved. Other such phenotypically silent substitutions are described in Bowie et al, and the references cited therein. The term "conservative substitution" is intended to mean that a substitution in which the amino acid residue is replaced with an amino acid residue having a similar side chain. These families are known in the art and include amino acids with basic side chains (e.g. lysine, arginine and histidine), acidic side chains (e.g. aspartic acid, glutamic acid), uncharged polar side chains (e.g., glycine, asparagines, glutamine, serine, threonine, tyrosine, cystein), non-polar side chains (e.g., alanine, valine, leucine, isoleucine, proline, phenylalanine, methionine, tryptophane), β-branched side chains (e.g., threonine, valine, isoleucine) and aromatic side chains (e.g., tyrosine, phenylalanine tryptophane, histidine).

A polypeptide with an amino acid sequence that is substantially homologous to the polypeptides of the present invention is defined as a polypeptide with an amico acid sequence with a degree of identity to the specified amino acid sequence of at least 50%, preferably at least 60%, more preferably at least 70%, still more preferably at least 80%, still more preferably at least 85%, still more preferably at least 90%, still more preferably at least 92%, still more preferably at least 94%, still more preferably at least 96%, still more preferably at least 98%, most preferably at least 99%.

In a second embodiment, variants of the amino acid sequences of the present inventions leading to an 'improved function' (defined herein as to an increased improvement of the desired properties defined in the first aspect) may be obtained by modifying the corresponding genes of the present invention. In the context of this invention such an 'improved function' is not limited to features like Kcat, Km, temperature optimum, half-life, turnover number, but may very well be a variant which is more resistant towards one or more of the β-lactam compounds and their intermediates (see Fig. 1 for an overview). Among such modifications are:
1. Error prone PCR to introduce random mutations, followed by a screening of obtained variants and isolating of variants with improved kinetic properties
2. Family shuffling of related variants of the genes of the present invention, followed by a screening of obtained variants and isolating of variants with "improved function" (these can be identified by the method of example 2)
3. Mutation of the specific residue, normally the target for feedback inhibition by β-lactam compounds or their intermediates

In the context of this invention 'improved genes' are variants of the genes of the present invention leading to an increased level of mRNA and/or protein, resulting in more enzyme activity which leads to leading to an 'improved function' (defined herein as to an increased improvement of the desired properties defined in the first aspect). These may be obtained by modifying the polynucleotide sequences of said genes. Among such modifications are:
1. Improving the codon usage in such a way that the codons are (optimally) adapted to the parent microbial host.
2. Improving the codon pair usage in such a way that the codons are (optimally)adapted to the parent microbial host
3. Addition of stabilizing sequences to the genomic information encoding the amino acid sequence(s) resulting in mRNA molecules with an increased half life

Preferred methods to isolate variants with improved function or increased levels of mRNA or protein are described in WO03010183 and WO0301311. Preferred methods to optimize the codon usage in parent microbial strains are described in PCT/EP2007/05594. Preferred methods to add stabilizing elements to the genes of the present invention are described in WO2005059149.

In a third embodiment, there is provided a polynucleotide or nucleic acid sequence comprising a DNA sequence encoding the polypeptides mentioned above. Table 1 shows how the SEQ ID No's of the genomic nucleotide sequences, the CDS sequences and the amino acid sequences are related to one another.

This may be an isolated polynucleotide of genomic, cDNA, RNA, semi-synthetic, synthetic origin, or any combinations thereof. In particular, specific sequences
- may have a genomic nucleotide sequence selected from the group consisting of SEQ ID No 1.
- may have a CDS nucleotide sequence selected from the group consisting of 2.

The genomic and CDS nucleotide sequences of the genes of the present invention are not limited to the sequences listed above but also comprise sequences that are "substantially homologous" to said sequences with the proviso that said genes encode an enzyme 'involved' in the production of β-lactam compounds and upon functional overexpresssion leads to an improvement of the desired properties defined in the first aspect.

A polynucleotide with a nucleotide sequence that is substantially homologous to the genes of the present invention is defined as a polynucleotide with a nucleotide sequence with a degree of identity to the specified nucleotide sequence of at least 70%, preferably at least 80%, more preferably at least 85%, still more preferably at least 90%, still more preferably at least 92%, still more preferably at least 94%, still more preferably at least 96%, still more preferably at least 98%, most preferably at least 99%.

Unless otherwise indicated, all nucleotide sequences determined by sequencing a DNA molecule herein are determined using an automated DNA sequencer and all amino acid sequences of polypeptides encoded by DNA molecules determined herein were predicted by translation of a DNA sequence determined as above. Therefore, for any DNA sequence determined by this automated approach, any nucleotide sequence determined may contain some errors. Nucleotide sequences determined by automation are typically at least about 90% identical, more typically at least about 95% to at least about 99.9% identical to the actual nucleotide sequence of the sequenced DNA molecule. The actual sequence can be more precisely determined by other approaches including manual DNA sequencing methods. As is also known in the art, a single insertion or deletion in a determined nucleotide sequence compared to the actual sequence will cause a frame shift in translation of the nucleotide sequence such that the predicted amino acid sequence encoded by a determined nucleotide sequence will be completely different from the amino acid sequence actually encoded by the sequenced DNA molecule, beginning at the point of such an insertion or deletion. The person skilled in the art is capable of identifying such erroneously identified bases and knows how to correct for such errors.

The polypeptides and the encoding nucleic acid sequences of the first aspect of the invention may be obtained from any cell, preferably from microbial strains capable of producing a β-lactam compound. These may be selected from the group consisting of a fungus, bacterium and yeast. Preferably the microbial strain of the present invention is a fungus, more preferably a filamentous fungus. A preferred filamentous fungus may be selected from the group consisting of *Aspergillus, Acremonium, Trichoderma* and *Penicillium.* More preferably the microbial strain of the present invention belongs to the species *Penicillium*, most preferably *Penicillium chrysogenum*. A preferred bacterium may be selected from the groups consisting of *Streptomyces, Nocardia,* or *Flavobacterium.* In a preferred embodiment, the microbial strain of the present invention capable of producing an N-adipoylated β-lactam compound belongs to the species *Penicillium*, most preferably is *Penicillium chrysogenum*, which has been transformed with a gene encoding an expandase, preferably the *Streptomyces clavuligerus cefE* gene, which enables the strain to produce adipoyl-7-ADCA when cultured in the presence of the precursor adipic acid. In another embodiment, the microbial strain of the present invention capable of producing an N-adipoylated β-lactam compound belongs to the species *Penicillium*, most preferably is *Penicillium chrysogenum* and which, in addition to an expandase gene, preferably the *Streptomyces clavuligerus cefE* gene, has been transformed with a hydroxylase gene, preferably the *Streptomyces clavuligerus cefF* gene, whose expression product converts the 3-methyl side chain of adipoyl-7-ADCA to 3-hydroxymethyl to give adipoyl-7-aminodeacetylcephalosporanic acid (adipoyl-7-ADAC). In another embodiment, the microbial strain of the present invention capable of producing an N-adipoylated β-lactam compound belongs to the species *Penicillium*, most preferably is *Penicillium chrysogenum*, has been transformed with a expandase/hydroxylase gene, preferably the *Acremonium chrysogenum cefEF* gene, whose expression product converts the 3-methyl side chain of adipoyl-7-ADCA to 3-hydroxymethyl, to give adipoyl-7-aminodeacetylcephalosporanic acid (adipoyl-7-ADAC). In another embodiment the microbial strain of the present invention capable of producing an N-adipoylated β-lactam compound belongs to the species *Penicillium*, most preferably is *Penicillium chrysogenum*, and in addition to the genes encoding expandase, preferably the *Streptomyces clavuligerus cefE* gene, and hydroxylase, preferably the *Streptomyces clavuligerus cefF* gene, is further transformed with an acetyltransferase gene, preferably the *Streptomyces clavuligerus cefG* gene, whose expression product (i.e. the acyltransferase) converts the 3-hydroxymethyl side chain to the 3-acetyloxymethyl side chain to give adipoyl-7-ACA. In a further embodiment the mutant microbial strain of the present invention capable of producing an N-adipoylated β-lactam compound belongs to the species *Penicillium*, most preferably is *Penicillium chrysogenum* and has been transformed with genes encoding an expandase, preferably the *Streptomyces clavuligerus cefE* gene, a hydroxylase, preferably the *Streptomyces clavuligerus cefF* gene and an O-carbamoyl transferase enzyme, preferably the *Streptomyces clavuligerus cmcH* gene, resulting in adipoyl-7-amino-3-carbamoyloxymethyl-3-cephem-4-carboxylic acid.

DNA sequences of the invention may be identified by hybridization. Nucleic acid molecules corresponding to variants (*e*.*g*. natural allelic variants) and homologues of the DNA of the invention can be isolated based on their homology to the nucleic acids disclosed herein using these nucleic acids or a suitable fragment thereof, as a hybridization probe according to standard hybridization techniques, preferably under highly stringent hybridization conditions. Alternatively, one could apply *in silico* screening through the available genome databases. "Stringency" of hybridization reactions is readily determinable by one of ordinary skill in the art. For additional details and explanation of stringency of hybridization reactions, see Ausubel et al. (1995, Current Protocols in Molecular Biology, Wiley Interscience Publishers).

The nucleic acid sequence may be isolated by *e*.*g*. screening a genomic or cDNA library of the microorganism in question. Once a nucleic acid sequence encoding a polypeptide having an activity according to the invention has been detected with *e*.*g*. a probe derived from one of the SEQ ID No 1 or 2, the sequence may be isolated or cloned by utilizing techniques which are known to those of ordinary skill in the art (see Sambrook et al., 1989, Molecular Cloning, A Laboratory Manual, 2d edition, Cold Spring Harbor, New York). The cloning of the nucleic acid sequences of the present invention from such (genomic) DNA can also be effected, *e*.*g*. by using methods based on polymerase chain reaction (PCR) or antibody screening of expression libraries to detect cloned DNA fragments with shared structural features (See, *e*.*g*., Innis et al., 1990, PCR: A Guide to Methods and Application, Academic Press, New York.).

The sequence information as provided herein should not be so narrowly construed as to require inclusion of erroneously identified bases. The specific sequences disclosed herein can be readily used to isolate the complete gene from Ascomycetes, in particular *Penicillium chrysogenum*, which in turn can easily be subjected to further sequence analyses thereby identifying sequencing errors.

In a fourth aspect, the invention provides a method for the construction of a mutant microbial strain capable of producing a β-lactam compound comprising the step of functionally overexpressing one or more genes which may have been selected by the method of the first aspect of the invention (the so-called 'positive gene(s)') and whereby the mutant microbial strain possesses one or more of the desired properties selected from the group consisting of
(i) an at least 10% higher concentration of the total β-lactam compound in the culture medium compared to the parent microbial strain; and/or
(ii) an at least 10% higher concentration of the desired β-lactam compound in the culture medium compared to the parent microbial strain; and/or
(iii) an at least 10% higher yield β-lactam / consumed sugar); and/or
(iv) an at least 5% higher yield in desired beta-lactam compound compared to undesired/total beta-lactam compound; and/or
(v) an at least 10% decreased consumption of side-chain precursor.

'Functional overexpression' or 'functionally overexpressing' or 'functionally overexpressed' is defined herein as the modification of the gene in such a way so as to obtain an activity of the enzyme encoded by the positive gene as compared to the activity of the enzyme in the parent strain of preferably more than 100%, more preferably more than 110%, more preferably more than 120%, more preferably more than 150%, more preferably more than 200%, more preferably more than 300%, more preferably more than 400%. Methods for the modification of the gene in order to obtain functional overexpression of the gene are known in the art and may include (but are not limited to): introduction of additional gene copies encoding host or heterologous proteins; over expression of host proteins from a strong promoter; modifying the transcriptional regulation of the genes controlling the genes involved in b-lactam production; mutation of critical amino acids leading to proteins with improved kinetic properties; mutations causing a increased half-life of the enzyme; modifying the mRNA molecule in such away that the mRNA half-life is increased; modifying the intracellular localization of the protein towards an organelle in which no products are present to inhibit its activity; functional inactivation of gene(s) and/or gene product(s) with a negative impact on these positive gene(s) and gene product(s); introduction of one or more copies of heterologous genes encoding enzymes mediating b-lactam resistance. Preferably overexpression is obtained by introducing additional gene copies or driving gene transcription from a strong promoter.

Functional overexpression of the gene of the invention in the mutant microbial strain of the invention is obtained with the use of nucleic acid constructs, e.g. expression constructs, which contain one or more of the selected genes, each operably linked to one or more control sequences, which direct the expression of the encoded polypeptide in a suitable expression host. The nucleic acid constructs may be on one DNA fragment, or, preferably, on separate fragments. Expression will be understood to include any step involved in the production of the polypeptide and may include transcription, post-transcriptional modification, translation, post-translational modification, and secretion. The term nucleic acid construct is synonymous with the term expression vector or cassette when the nucleic acid construct contains all the control sequences required for expression of a coding sequence in a particular host organism. The term "control sequences" is defined herein to include all components, which are necessary or advantageous for the expression of a polypeptide. Each control sequence may be native or foreign to the nucleic acid sequence encoding the polypeptide. Such control sequences may include, but are not limited to, a promoter, a leader, optimal translation initiation sequences (as described in Kozak, 1991, J. Biol. Chem. 266:19867-19870), a secretion signal sequence, a pro-peptide sequence, a polyadenylation sequence, a transcription terminator. At a minimum, the control sequences include a promoter, and transcriptional and translational stop signals. The term "operably linked" is defined herein as a configuration in which a control sequence is appropriately placed at a position relative to the coding sequence of the DNA sequence such that the control sequence directs the production of a polypeptide.

The control sequence may include an appropriate promoter sequence containing transcriptional control sequences. The promoter may be any nucleic acid sequence, which shows transcription regulatory activity in the cell including mutant, truncated, and hybrid promoters, and may be obtained from genes encoding extra cellular or intracellular polypeptides. The promoter may be either homologous or heterologous to the cell or to the polypeptide.

Preferred promoters for filamentous fungal cells are known in the art and can be, for example, the glucose-6-phosphate dehyrogenase *gpd*A promoters; protease promoters such as *pep*A, *pep*B, *pep*C; the glucoamylase *gla*A promoters; amylase *amy*A, *amy*B promoters; the catalase *cat*R or catA promoters; glucose oxidase *gox*C promoter; beta-galactosidase *lac*A promoter; alpha-glucosidase *agl*A promoter; translation elongation factor *tef*A promoter; xylanase promoters such as *xln*A, *xln*B, *xln*C, *xln*D; cellulase promoters such as *egl*A, *egl*B, *cbh*A; promoters of the β-lactam biosynthetic genes such as *pcbAB*, *pcbC, penDE;* inducible promoters such as *alcR* en *TETon*; promoters of transcriptional regulators such as *are*A, *cre*A, *xln*R, *pac*C, *prt*T, etc or any other, and can be found among others at the NCBI website (http://www.ncbi.nlm.nih.gov/entrez/).

In a preferred embodiment, the promoter may be derived from a gene, which is highly expressed (defined herein as the mRNA concentration with at least 0.5% (w/w) of the total cellular mRNA). In another preferred embodiment, the promoter may be derived from a gene, which is medium expressed (defined herein as the mRNA concentration with at least 0.01% until 0.5% (w/w) of the total cellular mRNA). In another preferred embodiment, the promoter may be derived from a gene, which is low expressed (defined herein as the mRNA concentration lower than 0.01% (w/w) of the total cellular mRNA).

In an even more preferred embodiment, Micro Array data is used to select genes, and thus promoters of those genes, that have a certain transcriptional level and regulation. In this way one can adapt the gene expression cassettes optimally to the conditions it should function in. Amongst the suitable promoters to be selected via this method are promoters of genes that have a constitutively very high transcript level or are induced under relevant (β-lactam) production conditions. Also, strong and/or constitutive promoters as isolated and/or disclosed by WO2007071399 can be considered to be suitable promoters.

Alternatively, one could clone random DNA fragments in front of the polynucleotides of this invention. Using an acetamide plate assay as disclosed by WO2007118836_one can easily screen for active promoters, as these should facilitate growth on acetamide as the sole nitrogen source. These DNA fragments can be derived from many sources, i.e. different species, PCR amplified, synthetically and the like.

The control sequence may also include a suitable transcription terminator sequence, a sequence recognized by a filamentous fungal cell to terminate transcription. The terminator sequence is operably linked to the 3' terminus of the nucleic acid sequence encoding the polypeptide. Any terminator, which is functional in the cell, may be used in the present invention. Preferred terminators for filamentous fungal cells are obtained from the genes encoding *Aspergillus oryzae* TAKA amylase, *Aspergillus niger* glucoamylase, *Aspergillus nidulans* anthranilate synthase, *Aspergillus niger* alpha-glucosidase, *trpC* gene, *Penicillium chrysogenum pcbC, Penicillium chrysogenum penDE* and *Fusarium oxysporum* trypsin-like protease. Alternatively, the original terminator of the gene of the invention is used.

The control sequence may also include a suitable leader sequence, a non-translated region of an mRNA, which is important for translation by the filamentous fungal cell. The leader sequence is operably linked to the 5' terminus of the nucleic acid sequence encoding the polypeptide. Any leader sequence, which is functional in the cell, may be used in the present invention. Preferred leaders for filamentous fungal cells are obtained from the genes encoding *Aspergillus oryzae* TAKA amylase and *Aspergillus nidulans* triose phosphate isomerase and *Aspergillus niger* glaA. Other preferred initiator sequences are isolated and/or disclosed by WO2006077258.

The control sequence may also include a polyadenylation sequence, a sequence which is operably linked to the 3' terminus of the nucleic acid sequence and which, when transcribed, is recognized by the filamentous fungal cell as a signal to add polyadenosine residues to transcribed mRNA. Any polyadenylation sequence, which is functional in the cell, may be used in the present invention. Preferred polyadenylation sequences for filamentous fungal cells are obtained from the genes encoding *Aspergillus oryzae* TAKA amylase, *Aspergillus niger* glucoamylase, *Aspergillus nidulans* anthranilate synthase, *Fusarium oxysporum* trypsin-like protease, *Penicillium chrysogenum pcbC, Penicillium chrysogenum penDE* and *Aspergillus niger* alpha-glucosidase. Alternatively, the original polyadenylation sequence of the gene of the invention is used.

The nucleic acid construct may be an expression vector. The expression vector may be any vector (e.g. a plasmid or virus), which can be conveniently subjected to recombinant DNA procedures and can bring about the expression of the nucleic acid sequence encoding the polypeptide. The choice of the vector will typically depend on the compatibility of the vector with the cell into which the vector is to be introduced. The vectors may be linear or closed circular plasmids.

The vector may be an autonomously replicating vector, i.e. a vector, which exists as an extrachromosomal entity, the replication of which is independent of chromosomal replication, e.g. a plasmid, an extrachromosomal element, a minichromosome, or an artificial chromosome. An autonomously maintained cloning vector for a filamentous fungus may comprise the AMA1-sequence (see e.g. Aleksenko and Clutterbuck (1997), Fungal Genet. Biol. 21: 373-397).

Alternatively, the vector may be one which, when introduced into the cell, is integrated into the genome and replicated together with the chromosome (s) into which it has been integrated. The integrative cloning vector may integrate at random or at a predetermined target locus in the chromosomes of the host cell. In a preferred embodiment of the invention, the integrative cloning vector comprises a DNA fragment, which is homologous to a DNA sequence in a predetermined target locus in the genome of host cell for targeting the integration of the cloning vector to this predetermined locus. In order to promote targeted integration, the cloning vector is preferably linearized prior to transformation of the host cell. Linearization is preferably performed such that at least one but preferably either end of the cloning vector is flanked by sequences homologous to the target locus. The length of the homologous sequences flanking the target locus is preferably at least at least 0.1 kb, even preferably at least 0.2kb, more preferably at least 0.5 kb, even more preferably at least 1 kb, most preferably at least 2 kb. Preferably, the parent microbial strains are modified for improved frequency of targeted DNA integration as described by WO05095624 and/or W02007115886.

The vector system may be a single vector or plasmid or two or more vectors or plasmids, which together contain the total DNA to be introduced into the genome of the host cell.

The DNA constructs may be used on an episomal vector. However in the present invention the constructs are preferably integrated in the genome of the host strain. Even more preferably the constructs are integrated at a predetermined site in the genome. Most preferably the constructs are integrated at the original genomic locus of the 'positive gene', which method is described in detail in the examples.

The microbial strain capable of producing a β-lactam compound may be selected from the group consisting of a fungus, bacterium or yeast as has been described hereinbefore under the first aspect of the invention.

Preferably, the invention provides a method for the construction of a mutant microbial strain capable of producing a β-lactam compound comprising the step of functionally overexpressing preferably one or more genes selected from Group 1 as defined hereinbefore or any gene which is at least 70%, more preferably at least 80%, more preferably at least 90%, more preferably at least 95%, more preferably at least 99% homologous to the any of the gene sequences of Group 1.

In a preferred embodiment, the invention provides a method for the construction of a mutant microbial strain capable of producing a β-lactam compound and whereby the mutant microbial strain gives an at least 10% higher concentration of the total β-lactam compound in the culture medium compared to the parent microbial strain, which method comprises the step of functionally overexpressing preferably one or more genes selected from Group 2 as defined hereinbefore or any gene which is at least 70%, more preferably at least 80%, more preferably at least 90%, more preferably at least 95%, more preferably at least 99% homologous to the any of the gene sequences of Group 2.

In another preferred embodiment of the method of the second aspect of the invention, the functional overexpressing of the one or more positive genes as defined hereinbefore, may be combined with a functional inactivation of one or more of the 'negative genes' as defined hereinbefore. This combination may lead to a further increase in one or more of the desired properties.

"Functionally inactivating" or "functionally inactive" or "functional inactivation" or "functionally inactivated" is defined herein as the inactivation of a gene which results in a residual activity of the encoded enzyme as compared to the activity in the parent strain of preferably less than 50%, more preferably less than 40%, more preferably less than 30%, more preferably less than 20%, more preferably less than 10%, more preferably less than 5%, more preferably less than 2%.

Methods for the modification of the gene in order to obtain the functionally inactive gene are known in the art and may include (but are not limited to): inactivation of the gene by base pair mutation resulting in a(n early) stop or frame shift; mutation of one or more codons which encode one or more a critical amino acids (such as the catalytic triad for hydrolases); mutations in the gene resulting in mutations in the amino acid sequence of the enzyme which lead to a decreased half-life of the enzyme; modifying the mRNA molecule in such away that the mRNA half-life is decreased; insertion of a second sequence (i.e. a selection marker gene) disturbing the open reading frame; a partial or complete removal of the gene; removal/mutation of the promoter of the gene; physical separation of promoter and gene by insertion of third DNA sequence (for example a selection marker gene); replacing the promoter of the gene by a regulatable promoter; using anti-sense DNA or comparable RNA inhibition methods to lower the effective amount of mRNA in the cell. Most preferably the gene may be made functionally inactive by deletion resulting in a total absence of the encoded polypeptide and hence enzyme activity.

In a fifth aspect, the invention provides a mutant microbial strain capable of producing a β-lactam compound **characterized in that** preferably one or more genes of the second aspect of the invention, preferably selected by the method of the first aspect of the invention, have been functionally overexpressed, preferably by the method according to the fourth aspect of the invention and whereby the mutant microbial strain possesses one or more of the desired properties selected from the group consisting of
i. an at least 10% higher concentration of the total β-lactam compound in the culture medium compared to the parent microbial strain; and/or
ii. an at least 10% higher concentration of the desired β-lactam compound in the culture medium compared to the parent microbial strain; and/or
iii. an at least 10% improved yield β-lactam / consumed sugar); and/or
iv. an at least 5% improved yield in desired beta-lactam compound compared to undesired/total beta-lactam compound; and/or
v. an at least 10% decreased consumption of side-chain precursor

Preferably, the invention provides a mutant microbial strain capable of producing a β-lactam compound **characterized in that** preferably one or more genes selected from Group 1 as defined hereinbefore or any gene which is at least 70%, more preferably at least 80%, more preferably at least 90%, more preferably at least 95%, more preferably at least 99% homologous to the any of the gene sequences of Group 1 are functionally overexpressed.

In another preferred embodiment the mutant microbial strain of the third aspect of the invention as defined hereinbefore in addition to the one or more functionally overexpressed gene(s) (positive genes) further comprises one or more functionally inactivated 'negative' genes as defined before.

It is defined herein that a sequence (sequence 1) is "substantially homologous" to another sequence (sequence 2) when sequence 1 possesses a degree of identity to sequence 2 of at least 75%, preferably at least 80%, preferably at least 85%, preferably at least 90%, preferably at least 95%, still more preferably at least 96%, still more preferably at least 97%, still more preferably at least 98% and most preferably at least 99%. This definition of "substantially homologous" applies to nucleotide sequences as well as to amino acid sequences.

In a sixth aspect, the invention provides a process for the production of a β-lactam compound comprising culturing the mutant microbial strain of the third aspect of the invention in a fermentation medium under conditions suitable for the production of β-lactam compound . Such conditions are well known to the skilled person.

### Figure legends

Figure 1 is an overview of the various enzymes and intermediates in β-lactam biosynthesis routes (i.e. penicillins, cephalosporins and cephamycins). The arrows represent the following enzymes: 1, L-α-aminoadipyl-L-cysteinyl-D-valine synthetase; 2, isopenicillin N synthase; 3, acyl-CoA:6-aminopenicillanic acid/isopenicillin N acyltransferase; 4, PenN epimerase; 5, deacetoxycephalosporin C synthase (expandase); 6, deacetylcephalosporin C hydroxylase; 7, 3'-hydroxymethylcephem-O-carbamoyltransferase; 8, O-carbamoyl-deacetylcephalosporin C hydroxylase; 9, methyltransferase; 10, acetyltransferase; 11, phenylacetyl-CoA ligase; 12, adipoyl-CoA-ligase. The capitals in brackets represent the following intermediates: [A], L-α-aminoadipic acid; [B], L-cysteine; [C], L-valine; [D], L-α-aminoadipoyl-L-cysteinyl-L-valine; [E], iso-penicillinN; [F], Penicillin G; [G], Penicillin N; [H], deacetoxycephalosporin C (DAOC); [I], deacetylcephalosporin C (DAC); [J], O-carbamoyl-DAC; [K], 7-α-hyroxy-OCDAC; [L], cephamycin C; [M], cephalosporin C; [N], adipoyl-6-aminopenicillinic acid (Ad-6-APA); [O], adipoyl-7-aminodeacetoxycephalosporanic acid (Ad-7-ADCA); [P], adipoyl-7-aminohydroxycephalosporanic acid (Ad-7-AHCA); [Q], adipoyl-7-amino-3-carbomoyloxymethyl-3-cephem-4-carboozylic acid (Ad-7-ACCA); [R], phenylacetic acid; [S]. phenylacetyl-CoA; [T], adipic acid; [U], adipoyl-CoA.

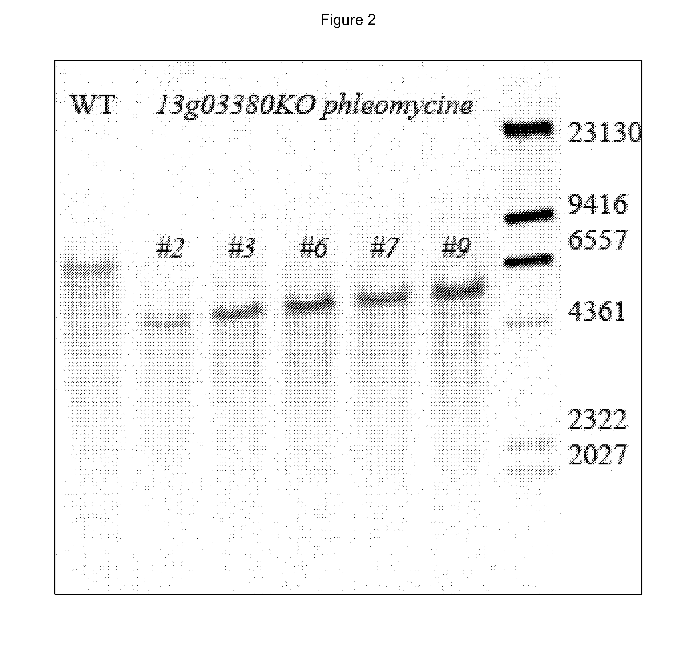

Figure 2 is a representation of the Southern Blot anlaysis of the patent microbial strain ans the mutant microbials strains wheren SEQ ID No 1 is deleted.

### General materials and methods

Standard DNA procedures were carried out as described elsewhere (Sambrook, J. et al., 1989, Molecular cloning: a laboratory manual, 2nd Ed., Cold Spring Harbor Laboratory Press, Cold Spring Harbor, New York) unless otherwise stated. DNA was amplified using the proofreading enzymes like Physion polymerase (Finnzymes). Restriction enzymes were from Fermentas. Fungal growth was performed in a mineral medium, containing (g/L): glucose (5); lactose (35); urea (4.5); (NH₄)₂SO₄ (1.1); Na₂SO₄ (2.9); KH₂PO₄ (5.2); K₂HPO₄ (4.8) and 10 mL/L of a trace element solution containing (in g/I): citric acid (150); FeSO₄.7H₂O (15); MgSO₄.7H₂O (150); H₃BO₃ (0.0075); CuSO₄.5H₂O (0.24); CoSO₄.7H₂O (0.375); ZnSO₄.7H₂O (5); MnSO₄.H₂O (2.28); CaCl₂.2H₂O (0.99); pH before sterilization 6.5.

### EXAMPLES

### Example 1

### Deletion of Penicillium chrysogenum gene Pc13g03380 (SEQ ID NO 1)

### Clone construction:

In order to prevent the transcription of gene Pc13g03380 (SEQ ID NO. 1) a selection marker gene encoding a protein mediating resistance to phleomycin was used to replace the open reading frame (ORF). To this end 2 kb regions up- and downstream of the ORF were PCR amplified using the oligonucleotides SEQ ID NO. 4 plus 5 and SEQ ID NO. 10 plus 11. Phusion Hot-Start Polymerase (Finnzymes) was used to amplify the fragments. The fragments obtained are 2055 basepairs (bp) in length (SEQ ID NO 6 and SEQ ID NO 12) and contain the so-called 'att' tails suitable for the Gateway cloning method (Invitrogen). The *ble* expression cassette (encoding a protein mediating resistance to phleomycin) was PCR amplified amplified using the oligonucleotides SEQ ID NO. 7 plus 8, yielding a 1538 bp fragment (SEQ ID NO. 9), also containing the so-called 'att' tails suitable for the Gateway cloning method (Invitrogen).

Constructs were made in Escherichia coli DH5α as a host strain using the Multisite Gateway® Three-Fragment Vector Construction Kit (Invitrogen) with different vectors. The upstream (also named 5' flanking) region of the gene was amplified with attB4 and attB1 sites and cloned into pDONRP4-P1R. The ble cassette was amplified with attB1 and attB2 sites and cloned into pDONR221. The downstream (also named 3' flanking) region of the gene was amplified with attB2 and attB3 sites and cloned into pDONRP2R-P3. The obtained plasmids were combined and cloned into pDESTR4-R3 destination vector of the Multisite Gateway® Three-Fragment Vector Construction Kit using LR Clonase II enzyme mix.

### Table 1.

Overview of genes identified in *Penicillium chrysogenum* by the method of the first aspect of the invention. The first column gives the unique Gene ID of each identified gene. Column 2 summarize the results (expressed as percentage relative to the control = parent strain) obtained upon functionally inactivating the corresponding gene. Column 2 gives the relative concentration of the total β-lactam compounds. Cells in column 2 which are highlighted (grey background and black font) contain values for each property which fulfil the criteria listed in step d(i) - d(v) of the method of the first aspect of the invention. Columns 3-9 summarize the SEQ ID Numbers for each identified gene. Column 3 gives the SEQ ID No for the genomic DNA sequence of each gene, column 4 the SEQ ID No for the corresponding Coding sequence (i.e. without the putative introns) and column 5 the SEQ ID No for the corresponding protein sequence. Columns 6-9 give the SEQ ID No for the oligonucleotides used as primers for each identified gene to obtain the fragments for functionally inactivating the respective gene as described in detail in example 1.

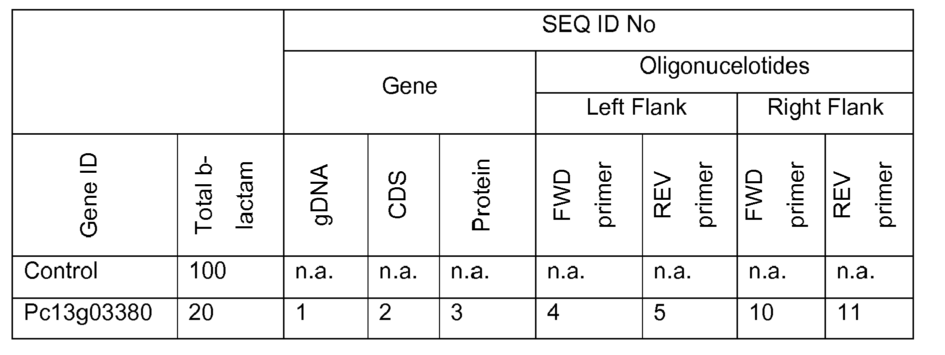

### Transformation:

The pDESTR4-R3 vector containing the deletion cassette for gene Pc13g03380 (containing the 5' flanking of Pc13g03380, the phleomycine resistance gene and the 3' flanking of Pc13g03380) was used to transform a derivative of *P. chrysogenum* strain DS17690 (S917) deposited at the Centraalbureau voor Schimmelcultures, Utrecht, The Netherlands on April 15, 2008 with deposition number CBS 122850 with the *hdfA* gene deleted (according to the method described in WO05095624). Protoplasts were isolated and transformation was performed according to Alvarez et al. (1987, Antimicrob. Agents Chemother. 31:1675-1682). Transformants were selected on phleomycine regeneration medium (for example YEPD, with 1.0 M saccharose and 50 µg/ml phleomycin). Selected colonies were transferred to medium to induce sporulation (for example YEPD, with 100 µg/ml phleomycin).

### PCR and Southern blot analysis:

Selected transformants were analyzed by PCR and Southern blot. Genomic DNA was isolated from 10 independent putative *P. chrysogenum* Δ*hdfA Δ*Pc13g03380 strains. Oligonucleotides used in PCR were designed to amplify a fragment of 664bp which confirm presence of phleomycine resistance gene (SEQ ID NO. 14 and 15) and a fragment of 431 bp to check for the presence of the Pc13g03380 gene (SEQ ID NO. 13 and 14). Genomic DNA isolated from *P. chrysogenum* Δ*hdfA* and the pDESTR4-R3 vector containing the deletion cassette for gene Pc13g03380 were used as controls. Five of the 10 samples analysed showed a clear band of -650 bp in the *ble* reaction, indicating the correct presence of the selectionmarker gene, while no band in the Pc13g03380 reaction, suggested a correct deletion.

To confirm the deletion a Southern blot with genomic DNA of the five putative ΔPc13g03380 strains was performed. As control, genomic DNA isolated from *P*. *chrysogenum* Δ*hdfA* was used. DNA was digested with Xhol overnight. The PCR amplified 3' flanking region (using SEQ ID NO. 13 and 14) was used as a probe. A correct deletion should lead to a band of 5119 bp while the control should give a band of 7418bp. As depicted in Fig. 2 in all five candidates Pc13g03380 is properly deleted.

### Example 2

### Deletion of the Penicillium chrysogenum Pc13g03380 (SEQ ID NO 1) leads to a complete block of b-lactam biosynthesis

Spores of *P. chrysogenum* Δ*hdfA* and *P. chrysogenum* Δ*hdfA* ΔPc13g03380 were used to inoculate liquid medium with phenylacetic acid or phenoxyacetic acid as side chain precursor. Cultivation was for 7 days, 280 rpm at 25 C Celsius. Afterwards the supernatant was analysed for the b-lactam compounds produced (see table 1). To this end, cells were removed by centrifugation and 1 ml of the supernatant was used for NMR analysis. Quantitative ¹H NMR experiments were performed at 600 MHz on a Bruker Avance 600 spectrometer. To a known quantity of filtrate, a known quantity of internal standard (maleic acid), dissolved in phosphate buffer was added prior to lyophilisation. The residue was dissolved in D₂O and measured at 300°K. The delay between scans (30 s) was more than 5 times T₁ of all compounds, so the ratio between the integrals of the compounds of interest and the integral of the internal standard is an exact measure for the quantity of the penicillins, intermediates (6-APA and IsopenicillinN), degradation products (8-HPA), remaining sugar and remaining side-chain (adipate).

As a control the parent strain Δ*hdfA* derivative of *P. chrysogenum* DS17690 (S917) deposited at the Centraalbureau voor Schimmelcultures, Utrecht, The Netherlands on April 15, 2008 with deposition number CBS 122850 was cultivated and processed in the same way. The total β-lactam relative to the value of the control strain is significantly decreased as a consequence of the functional inactivation of gene Pc13g03380, indicatived it is 'involved' in the production of b-lactam compounds as a 'positive gene'..

### Example 3

### Functional overexpression of specific Penicillium chrysogenum genes

In order to functionally overexpress the genes and encoded proteins identified as 'positive genes' (see examples 1 and 2), a strong promoter is inserted between the original promoter and the open reading frame (ORF) of selected 'positive genes'. Basically the same PCR-amplified fragments (i.e. promoter and ORF) of example 1 are used. To drive over expression the ORF is cloned in a variant vector of pSTamdSR, which contains a strong promoter downstream of the *trpC* terminator. Further steps (i.e. cloning, 2^{nd} PCR, transformation, selection of transformants) are as in example 2.

## Claims

1. A method for the identification of one or more genes of a parent microbial strain capable of producing a β-lactam compound comprising the steps of
a. Functionally inactivating the one or more genes in the parent microbial strain thereby generating one or more mutant microbial strains whereby each mutant microbial strain has at least one functionally inactivated gene;
b. Culturing the parent microbial strain and the mutant microbial strains obtained in step (a) in a medium under conditions that allow production of a β-lactam compound;
c. Measuring the concentration of the β-lactam compound in the culture medium of the parent microbial strain and the one or more mutant microbial strains;
d. Selecting the mutant microbial strains which possess one or more of the desired properties selected from the group consisting of
(i) an at least 10% lower concentration of the total β-lactam compound in the culture medium compared to the parent microbial strain; and/or
(ii) an at least 10% lower concentration of the desired β-lactam compound in the culture medium compared to the parent microbial strain; and/or
(iii) an at least 10% lower yield (β-lactam / consumed sugar); and/or
(iv) an at least 5% lower yield in desired beta-lactam compound compared to undesired/total beta-lactam compound; and/or
(v) an at least 10% increased consumption of side-chain precursor.
e. Optionally identifying the gene(s) in the selected mutant microbial strain
f. Optionally repeating step a. - d. whereby a selected mutant strain obtained in step d. is used as the parent strain in step a.

2. A gene identifiable by the method of claim 1.

3. A gene according to claim 2 selected from group 1 consisting of gDNA sequences with SEQ ID No 1.

4. A polypeptide encoded by the gene of any of claim 2 or 3.

5. A polypeptide according to claim 4 selected from the group consisting of protein sequences with SEQ ID No 3.

6. A method for the construction of a mutant microbial strain capable of producing a β-lactam compound comprising the step of functionally overexpressing one or more genes as defined in claim 2 and 3 and whereby the mutant microbial strain possesses one or more of the desired properties selected from the group consisting of
i. an at least 10% higher concentration of the total β-lactam compound in the culture medium compared to the parent microbial strain; and/or
ii. an at least 10% higher concentration of the desired β-lactam compound in the culture medium compared to the parent microbial strain; and/or
iii. an at least 10% improved yield (β-lactam / consumed sugar); and/or
iv. an at least 5% improved yield in desired beta-lactam compound compared to undesired/total beta-lactam compound; and/or
v. an at least 10% decreased consumption of side-chain precursor

7. The method according to claim 6 comprising the step of functionally overexpressing preferably one or more genes as defined in claim 3.

8. A mutant microbial strain capable of producing a β-lactam compound **characterized in that** one or more genes, preferably the genes as defined in claim 2 and 3, are functionally overexpressed preferably according to the method of claim 6 and whereby the mutant microbial strain possesses one or more of the desired properties selected from the group consisting of
i. an at least 10% higher concentration of the total β-lactam compound in the culture medium compared to the parent microbial strain; and/or
ii. an at least 10% higher concentration of the desired β-lactam compound in the culture medium compared to the parent microbial strain; and/or
iii. an at least 10% improved yield (β-lactam / consumed sugar); and/or
iv. an at least 5% improved yield in desired beta-lactam compound compared to undesired/total beta-lactam compound; and/or
v. an at least 10% decreased consumption of side-chain precursor

9. The mutant microbial strain of claim 8 **characterized in that** preferably one or more genes as defined in claim 3 are functionally overexpressed.

10. Process for the production of a β-lactam compound using the mutant microbial strain of claim 8 or 9.

11. Use of the mutant microbial strain of claim 8 or 9 for the production of a β-lactam compound.


SEQUENCE LISTING

<110>  DSM IP Assets B.V.

<120>  BETA-LACTAM PRODUCING STRAINS

<130>  26715EP/P0/

<160>  15

<170>  PatentIn version 3.5

<210>  1
<211>  1174
<212>  DNA
<213>  Penicillium chrysogenum

<400>  1
atggccggtc aatccaaacc tgcgctgtcg ccatggggca gcgccgtggc aggcgcgact      60

ggtgccgtcc ttgcaaacgc gatggtgtac ccacttgact tgtgaggacc cagataaaac     120

ttatcagatc aaatcccgct gactttaatg cccccgatcc gtagagtcaa gacgaaacta     180

caagtacaag tcaaagaaaa gaacggaacc gtgcggcccg ataacctcga acattatgaa     240

tcgaccatgg atgctatcac caagatcgtg gagaaggagg gctataatgg tctgtactcg     300

ggaatggctg gagcccttct aggcgtcgcc tcaacaaact tcgcatactt ctattggtac     360

agcgtcgtaa gaacgctcta catggcttct gccaagccga cacaggcccc cggcaccgca     420

attgagctct ccctgggcgc cgtctccggc gccgtggctc agcttttcac tatcccggtg     480

gccgttatca caacccgaca gcagacacag ccgaagggcg agaagaaggg gttgatcgag     540

acgggtcggg aagtggtcga cagcgaggac ggctggagcg gcctgtggcg cggcctgaag     600

gctagcttga tcctggtcgt taacccggcc atcacctatg gcgcatacca gcgtctaaag     660

gatgttatct tcccagggaa gaacaacctc aagccctggg aggctttccg tgagtagtgg     720

caattctccg ggtcaacaat ggcttcctaa ttattatttt agtgcttggt gccatgtcca     780

aggctttggc cacgatcgca acccagcctc tgattgtcgc caaggttgga ctgcagtctc     840

gcccaccgcc gagccggaat ggcaagccct tcaagacctt tggtgaggtc atgaaacaca     900

ttgttgataa tgagggtctc ttttccctct tcaagggtat cggacctcag attctgaaag     960

gtcttttggt ccaaggtctg ctcatgatga ccaaggagag gtatgtttct gtttccaaat    1020

taccatctgg ttgtactaac ctttgagaag gatggagctc cttttcatcg tcctgttcgc    1080

ctacctcaag aacatcaaga aggacaaatt gcgcaaggca gtggatgctg cagctgaaaa    1140

ggccaagacc agcatgcctg ctaccctgaa ataa                                1174

<210>  2
<211>  1008
<212>  DNA
<213>  Penicillium chrysogenum

<400>  2
atggccggtc aatccaaacc tgcgctgtcg ccatggggca gcgccgtggc aggcgcgact      60

ggtgccgtcc ttgcaaacgc gatggtgtac ccacttgact tagtcaagac gaaactacaa     120

gtacaagtca aagaaaagaa cggaaccgtg cggcccgata acctcgaaca ttatgaatcg     180

accatggatg ctatcaccaa gatcgtggag aaggagggct ataatggtct gtactcggga     240

atggctggag cccttctagg cgtcgcctca acaaacttcg catacttcta ttggtacagc     300

gtcgtaagaa cgctctacat ggcttctgcc aagccgacac aggcccccgg caccgcaatt     360

gagctctccc tgggcgccgt ctccggcgcc gtggctcagc ttttcactat cccggtggcc     420

gttatcacaa cccgacagca gacacagccg aagggcgaga agaaggggtt gatcgagacg     480

ggtcgggaag tggtcgacag cgaggacggc tggagcggcc tgtggcgcgg cctgaaggct     540

agcttgatcc tggtcgttaa cccggccatc acctatggcg cataccagcg tctaaaggat     600

gttatcttcc cagggaagaa caacctcaag ccctgggagg ctttcctgct tggtgccatg     660

tccaaggctt tggccacgat cgcaacccag cctctgattg tcgccaaggt tggactgcag     720

tctcgcccac cgccgagccg gaatggcaag cccttcaaga cctttggtga ggtcatgaaa     780

cacattgttg ataatgaggg tctcttttcc ctcttcaagg gtatcggacc tcagattctg     840

aaaggtcttt tggtccaagg tctgctcatg atgaccaagg agaggatgga gctccttttc     900

atcgtcctgt tcgcctacct caagaacatc aagaaggaca aattgcgcaa ggcagtggat     960

gctgcagctg aaaaggccaa gaccagcatg cctgctaccc tgaaataa                 1008

<210>  3
<211>  335
<212>  PRT
<213>  Penicillium chrysogenum

<400>  3

Met Ala Gly Gln Ser Lys Pro Ala Leu Ser Pro Trp Gly Ser Ala Val
1               5                   10                  15

Ala Gly Ala Thr Gly Ala Val Leu Ala Asn Ala Met Val Tyr Pro Leu
            20                  25                  30

Asp Leu Val Lys Thr Lys Leu Gln Val Gln Val Lys Glu Lys Asn Gly
        35                  40                  45

Thr Val Arg Pro Asp Asn Leu Glu His Tyr Glu Ser Thr Met Asp Ala
    50                  55                  60

Ile Thr Lys Ile Val Glu Lys Glu Gly Tyr Asn Gly Leu Tyr Ser Gly
65                  70                  75                  80

Met Ala Gly Ala Leu Leu Gly Val Ala Ser Thr Asn Phe Ala Tyr Phe
                85                  90                  95

Tyr Trp Tyr Ser Val Val Arg Thr Leu Tyr Met Ala Ser Ala Lys Pro
            100                 105                 110

Thr Gln Ala Pro Gly Thr Ala Ile Glu Leu Ser Leu Gly Ala Val Ser
        115                 120                 125

Gly Ala Val Ala Gln Leu Phe Thr Ile Pro Val Ala Val Ile Thr Thr
    130                 135                 140

Arg Gln Gln Thr Gln Pro Lys Gly Glu Lys Lys Gly Leu Ile Glu Thr
145                 150                 155                 160

Gly Arg Glu Val Val Asp Ser Glu Asp Gly Trp Ser Gly Leu Trp Arg
                165                 170                 175

Gly Leu Lys Ala Ser Leu Ile Leu Val Val Asn Pro Ala Ile Thr Tyr
            180                 185                 190

Gly Ala Tyr Gln Arg Leu Lys Asp Val Ile Phe Pro Gly Lys Asn Asn
        195                 200                 205

Leu Lys Pro Trp Glu Ala Phe Leu Leu Gly Ala Met Ser Lys Ala Leu
    210                 215                 220

Ala Thr Ile Ala Thr Gln Pro Leu Ile Val Ala Lys Val Gly Leu Gln
225                 230                 235                 240

Ser Arg Pro Pro Pro Ser Arg Asn Gly Lys Pro Phe Lys Thr Phe Gly
                245                 250                 255

Glu Val Met Lys His Ile Val Asp Asn Glu Gly Leu Phe Ser Leu Phe
            260                 265                 270

Lys Gly Ile Gly Pro Gln Ile Leu Lys Gly Leu Leu Val Gln Gly Leu
        275                 280                 285

Leu Met Met Thr Lys Glu Arg Met Glu Leu Leu Phe Ile Val Leu Phe
    290                 295                 300

Ala Tyr Leu Lys Asn Ile Lys Lys Asp Lys Leu Arg Lys Ala Val Asp
305                 310                 315                 320

Ala Ala Ala Glu Lys Ala Lys Thr Ser Met Pro Ala Thr Leu Lys
                325                 330                 335

<210>  4
<211>  47
<212>  DNA
<213>  Artificial Sequence

<220>
<223>  Synthetic oligonucleotide

<400>  4
ggggacaact ttgtatagaa aagttgaatt ctgaagaact tgttcac                    47

<210>  5
<211>  48
<212>  DNA
<213>  Artificial Sequence

<220>
<223>  Synthetic oligonucleotide

<400>  5
ggggactgct tttttgtaca aacttgcgat gtacgttaac ggtattcg                   48

<210>  6
<211>  2055
<212>  DNA
<213>  Penicillium chrysogenum

<400>  6
ggggacaact ttgtatagaa aagttgaatt ctgaagaact tgttcacagt ccatccaaaa      60

cgtgcctggc gaaaggggag gtttatcatt caggccgagt gagccgagat ctgccgtaag     120

actgctttgt atatggggtt ctgcaccggc ccacgggttc ttcaaaagaa actgacgacg     180

accctggact tccttcaggt ccaggatagc atagtcgtgc tcgctaacta gccccagtcc     240

ttgttgttcc agttcagtca atttcccagt accgatagtt aaaaccacat caccatgcca 300

gaaggcgctg tataaacgcc cccagatctc atcagaggtc gaatcgtcgt gatgtaaaaa 360

gacttgttcc ggaatccaac cagtaagcac ccaaaggtcg gtccctgagt tacttcctgg 420

gaaatcatag cctccacgga ccttcaagta ggccttctcc acaagagcag gccacaaaaa 480

gttcgagttg ttgcgatcga tcacatgaag tgagcgtgta gtcttcgatg atggcaaccg 540

atcatctatc actaccttcc ggaaagaccc attgaagtag aagcgaaata tgtacttccc 600

agaatgcgaa agtattgggt tcttatcctc actgcatggg tacagtgtcg gtgatgcatg 660

ctgttcgaat tagctctggg actcatcgac gtgggatcaa attggtccat acctgcccta 720

gacctcgttc cgatcgagca gttgtggcgc aaaggcttgc gacgactgaa caatctgtta 780

agacatcctg cacgaggtct gtttcccccg aagccgacat agctggatta attatatcat 840

acggctgctt ggccgtattc attgtctgca acagatcatg tggccgtttc cagcctgcga 900

aaatttccct ctggcattca gagaggtgta aatcaggcgt atccctatac ccggttagaa 960

tagatcagca tttacgccat gttctccaca tacgtaaaaa gctgcccttt atatacaaat 1020

tcatcctgag gcggtgcgcg actccatggt ggaaagatat aaccgttgag tttggcaccc 1080

tcaaggatga tgatctcttc acgggtggtg agcttccgcg tggatgccgg cggccgtagg 1140

ctagggaagg ctttctgggg ccccgcacga gcagccgact gccagtcagt ggcggccttg 1200

atcttctcag ctttggatat caattccttg catttggcgt ctagtagctt tttgtcgtcg 1260

gcgggcgcca ggcgcagcgc tttcatgtaa tgctcggcgg cattgatagc tgcctccaat 1320

gccgctttct tggaggtcgc tgcggctatc gcctcttcag cgctctatca aatcattagc 1380

ttccttaaga tgtaagtgta aacagaggca aaattacccg tgcttgagat ataagggatt 1440

gtgtggatgc aggtctagac atacttgcgt tgactagtcg tggaggtaat cagatcgaac 1500

agcaagtaaa ttaccggatt cggctgagcc gagagaaggc cactttaatt ccggtgaata 1560

ttccgagacg tggatttgta gagaccaaca tattgatgat tattattgta ttcaattcta 1620

taactataca aatcgaaaat aacaaggaac taatattcag agaaaaaaaa aaagaaataa 1680

aaaaagtaaa cacatggaat gcaagtcatg acataagctc atactgacct caccccgaag 1740

tggacctcgg ctcgttttaa ggtgagaacg ccgaggtgca tttgcatttg agaccggctc 1800

atgagctcgg tctctttttt cattattcgg ttgacttcgg tatgaccata tctgtgtgtg 1860

tttgtgtttc cccattatac tctctcttcc atttcacctt ctccgctgtt cggagccaag 1920

cagtgtagca gccccgccca atatttctcc tgtcgcccga agcgaaccct ctccgcgggt 1980

catattctta attgttgcgc tcactttcga ataccgttaa cgtacatcgc aagtttgtac 2040

aaaaaagcag tcccc 2055

<210> 7
<211> 54
<212> DNA
<213> Artificial Sequence

<220>
<223> Synthetic oligonucleotide

<400> 7
ggggacaagt ttgtacaaaa aagcaggctc ccctcgaggt cgactacatg tatc 54

<210> 8
<211> 55
<212> DNA
<213> Artificial Sequence

<220>
<223> Synthetic oligonucleotide

<400> 8
ggggaccact ttgtacaaga aagctgggtt tgcaaattaa agccttcgag cgtcc 55

<210> 9
<211> 1538
<212> DNA
<213> Streptomyces hindustanus

<400> 9
ggggacaagt ttgtacaaaa aagcaggctc ccctcgaggt cgactacatg tatctgcatg 60

ttgcatcggg aaatcccacc acagggacag ccaagcggcc ccgcgacttg gcagtgggca 120

aactacgccc gattctggtg ccaagaaccg agaagaatga gacagaccca cgttgcactc 180

taaccggatg ctatcgactt acggtggctg aagattcaac acgctgcaac gagagccaag 240

gtggtccgga cattttctac gtgccggttt accttggaac atcgccgtcg ttgagtgcac 300

gttgcctact ctctcgtggc ttggctgggc ccacgagccc gattgactcg acggtgttac 360

ttgggtatct atggccccgt tttctggcac ggtaatgata agtacttact agtcttcgag 420

cgagcggggg agtgttgctc tgcccgagca tcaacgattg gcctgatcgc accgtctgca 480

aatgccacgg tgcggaccga ctgaaatctc agaccaccaa agaccctccg acttcgagat 540

acggttacta attttacact ggctccagcg gccccatcca gtaagcatct gggctgcaag 600

cgtataatgt ctccaggttg tctcagcata aacaccccgc ccccgctcag gcacacagga 660

agagagctca ggtcgtttcc attgcgtcca tactcttcac tcattgtcat ctgcaggaga 720

acttcccctg tccctttgcc aagccctctc ttcgtcgttg tccacgcctt caagttttca 780

ccattatttt tctagacacc atggccaagt tgaccagtgc cgttccggtg ctcaccgcgc 840

gcgacgtcgc cggagcggtc gagttctgga ccgaccggct cgggttctcc cgggacttcg 900

tggaggacga cttcgccggt gtggtccggg acgacgtgac cctgttcatc agcgcggtcc 960

aggaccaggt ggtgccggac aacaccctgg cctgggtgtg ggtgcgcggc ctggacgagc 1020

tgtacgccga gtggtcggag gtcgtgtcca cgaacttccg ggacgcctcc gggccggcca 1080

tgaccgagat cggcgagcag ccgtgggggc gggagttcgc cctgcgcgac ccggccggca 1140

actgcgtgca cttcgtggcc gaggagcagg actgaccgac gccgaccaac accgccggtc 1200

cgacgcggcc cgacgggtcc gaggcctcgg agatccgtcc cccttttcct ttgtcgatat 1260

catgtaatta gttatgtcac gcttacattc acgccctccc cccacatccg ctctaaccga 1320

aaaggaagga gttagacaac ctgaagtcta ggtccctatt tattttttta tagttatgtt 1380

agtattaaga acgttattta tatttcaaat ttttcttttt tttctgtaca gacgcgtgta 1440

cgcatgtaac attatactga aaaccttgct tgagaaggtt ttgggacgct cgaaggcttt 1500

aatttgcaaa cccagctttc ttgtacaaag tggtcccc 1538

<210> 10
<211> 51
<212> DNA
<213> Artificial Sequence

<220>
<223> Synthetic oligonucleotide

<400> 10
ggggacagct ttcttgtaca aagtggtctt ttcttatagt cagacggagg g 51

<210> 11
<211> 50
<212> DNA
<213> Artificial Sequence

<220>
<223> Synthetic oligonucleotide

<400> 11
ggggacaact ttgtataata aagttgaatt cataaagaaa agaaaaaagc 50

<210>  12
<211>  2055
<212>  DNA
<213>  Penicillium chrysogenum

<400>  12
ggggacagct ttcttgtaca aagtggtctt ttcttatagt cagacggagg gattttgagt      60

ttccccttgg attctgaatt gtgtttacct aactggcgcg tcggtatgcc gttggcaatt     120

ctggtggatt ttgaacagtc tagacatttc tcggcatttt cttgctggaa acacttgata     180

tgatagaacc cggtgtttgt agcttttcaa tatcattacg gatctgtata tggccatggg     240

cattccttac ttcctttagc accaatgcac tgcttctatc agcacgctat gtagttaacc     300

atccctggtt atacataaat atacaaatga aaatatgaaa atgtcgacgg acgtagagga     360

cctcaacctt ggaaccttgg aattatagtc taattagtac ctaccttggc ggctctgcca     420

tgtcaaatcc gaatgggcct tagttgcttt tcttttactt catgacagaa atcaggtgta     480

tgatgaatgt atgtcaattt cacaatgata aaattctcta actaactatg taacaaaaat     540

ggtctggttt tgacaatctg ttcagaccct ctctcacttt gctctcactt ccagcttcgt     600

ttgtcccctc catcacctac ggtcctatcg ttgtctctgc atgatctgta actatatagg     660

aatccggggt tcgtgatgct atagatgatg aaaacggggt ctgttccggt ataaaaggtt     720

ggttttggat ggtatgggag gatggagagg ttggtaaggt tggtgatggt gaggttggtg     780

gagaaggtag gggtatatcc gctaggaaag atcctggcat atagtctctg tttgctgttt     840

gcctttctag cggtctgatc gatgccgggg tggtgtgaga tcagatggtt tttggatgac     900

aaatggtgtt tgctgcctat caccttcctc tcttctctca cttctctcac ttctctcact     960

tcgcatctct ctctctgcat ctgcatctgc agcttcatct gcatctgcat ctatatacgg    1020

atcctgacct tattgtgtat ggtgagtatc atcatagaga gcaatgcggg ctattgctaa    1080

cgtcatctac atctgggtgc taacatcaga ccgagcttca tcccatataa gaactgctgg    1140

caggagagat cccatttctt tcgtactaac tgcttcttca tacggctttt ctcccgctct    1200

atatcgccag ttttcatcaa acacagcgtc cctgacgaca tctatccttc tatgttggcc    1260

tcctttctgt gggaaccaga tccgatatag gtttttagag acatacccta ctaaccatcc    1320

aatctctact ctaggagcca ttttgtcaga ttgggttctt ttcgaatcca ggatccggga    1380

gtacgccaag cagctataca ccttaggcta aggtttgaaa ggttgatacg ctgatccttg    1440

atcccatctg gtgcagccag tttcatcagc tccatccatg gtattatccg atttccattg    1500

gtgagctttg tcggcatccg atttaagata tatgctgacg tgtataccgc ttctggccag    1560

agcgttttcg gcagatctgg gttttcatta accagagacc ttgccatacg cactatcaca     1620

cccccggacc tttcggctgg tccgttcatt tctggagctt caggtggtga atgctcaata     1680

tttgtacttc tgtttcatta ttgcgtacct gagtgcaaag agccgaggag ccagtgacgc     1740

cgttgcgaaa aaggctgaag ctacgcgttt ttggccctcc gccggcgaat accttaatcg     1800

gtcaacactg gttacattag cccgttgtgt ttcaggatcg gaactccctg aatcccttgt     1860

tcaagacaag acgtttgact ccgatgatac ctccactcgt gacgctgacg ctggaactgt     1920

gcttggagct gatgtccgtg acgcaattct tgccatgaag aatctctaaa tcattgctat     1980

gctaagccac ggatgagtca aatttgcttt tttcttttct ttatgaattc aactttatta     2040

tacaaagttg tcccc                                                      2055

<210>  13
<211>  22
<212>  DNA
<213>  Artificial Sequence

<220>
<223>  Synthetic oligonucleotide

<400>  13
gtactaacct ttgagaagga tg                                              22

<210>  14
<211>  22
<212>  DNA
<213>  Artificial Sequence

<220>
<223>  Synthetic oligonucleotide

<400>  14
ctacatagcg tgctgataga ag                                              22

<210>  15
<211>  21
<212>  DNA
<213>  Artificial Sequence

<220>
<223>  Synthetic oligonucleotide

<400>  15
gcaactgcgt gcacttcgtg g                                               21